# EUROPEAN PATENT APPLICATION

(11) **EP 3 892 296 A1**
(43) Date of publication of application: **13.10.2021**
(21) Application number: 20168554.2
(22) Date of filing: 07.04.2020
(51) Int. Cl.: A61K 39/12

(54) **IMMUNOGENIC COMPOSITION COMPRISING AN ANTIGENIC MOIETY AND A LIPOSOMAL FORMULATION, METHOD OF PRODUCING THE COMPOSITION, THE COMPOSITION FOR USE AS A MEDICAMENT, IN PARTICULAR FOR USE AS A VACCINE**

(71) Applicant: InnoMedica Holding AG, 6300 Zug (CH)
(72) Inventor: Halbherr, Stéfan Jonathan, 3011 Bern (CH); Peitsch, Camille, 3012 Bern (CH)
(74) Representative: Hepp Wenger Ryffel AG

(57) **Abstract**

The present invention concerns an immunogenic composition comprising (a) an antigenic moiety, preferably an antigenic moiety being or comprising an amino acid sequence corresponding to a surface protein domain of SARS-CoV-2 virus; and (b) a liposomal formulation as an adjuvant. More specifically, the antigenic moiety preferably is either the receptor binding domain RBD of Spike protein S of SARS-CoV-2 virus, or the HR domain of S₂ subunit of spike protein S of SARS-CoV-2 virus; or an immunogenic fragment thereof.

SEQUENCE LISTING

<110>  Innomedica Holding AG

<120>  Immunogenic composition comprising an antigenic moiety and a
       liposomal formulation

<130>  PIMH004EP

<160>  8

<170>  PatentIn version 3.5

<210>  1
<211>  192
<212>  PRT
<213>  SARS-CoV-2 virus

<400>  1

Pro Asn Ile Thr Asn Leu Cys Pro Phe Gly Glu Val Phe Asn Ala Thr
1               5                   10                  15

Arg Phe Ala Ser Val Tyr Ala Trp Asn Arg Lys Arg Ile Ser Asn Cys
            20                  25                  30

Val Ala Asp Tyr Ser Val Leu Tyr Asn Ser Ala Ser Phe Ser Thr Phe
        35                  40                  45

Lys Cys Tyr Gly Val Ser Pro Thr Lys Leu Asn Asp Leu Cys Phe Thr
    50                  55                  60

Asn Val Tyr Ala Asp Ser Phe Val Ile Arg Gly Asp Glu Val Arg Gln
65                  70                  75                  80

Ile Ala Pro Gly Gln Thr Gly Lys Ile Ala Asp Tyr Asn Tyr Lys Leu
                85                  90                  95

Pro Asp Asp Phe Thr Gly Cys Val Ile Ala Trp Asn Ser Asn Asn Leu
            100                 105                 110

Asp Ser Lys Val Gly Gly Asn Tyr Asn Tyr Leu Tyr Arg Leu Phe Arg
        115                 120                 125

Lys Ser Asn Leu Lys Pro Phe Glu Arg Asp Ile Ser Thr Glu Ile Tyr
    130                 135                 140

Gln Ala Gly Ser Thr Pro Cys Asn Gly Val Glu Gly Phe Asn Cys Tyr
145                 150                 155                 160

Phe Pro Leu Gln Ser Tyr Gly Phe Gln Pro Thr Asn Gly Val Gly Tyr
                165                 170                 175

Gln Pro Tyr Arg Val Val Val Leu Ser Phe Glu Leu Leu His Ala Pro
            180                 185                 190

<210> 2
<211> 192
<212> PRT
<213> SARS-CoV-2 virus

<400> 2

Pro Asn Ile Thr Asn Leu Ser Pro Phe Gly Glu Val Phe Asn Ala Thr
1               5                   10                  15

Arg Phe Ala Ser Val Tyr Ala Trp Asn Arg Lys Arg Ile Ser Asn Ser
            20                  25                  30

Val Ala Asp Tyr Ser Val Leu Tyr Asn Ser Ala Ser Phe Ser Thr Phe
        35                  40                  45

Lys Ser Tyr Gly Val Ser Pro Thr Lys Leu Asn Asp Leu Ser Phe Thr
    50                  55                  60

Asn Val Tyr Ala Asp Ser Phe Val Ile Arg Gly Asp Glu Val Arg Gln
65                  70                  75                  80

Ile Ala Pro Gly Gln Thr Gly Lys Ile Ala Asp Tyr Asn Tyr Lys Leu
                85                  90                  95

Pro Asp Asp Phe Thr Gly Ser Val Ile Ala Trp Asn Ser Asn Asn Leu
            100                 105                 110

Asp Ser Lys Val Gly Gly Asn Tyr Asn Tyr Leu Tyr Arg Leu Phe Arg
        115                 120                 125

Lys Ser Asn Leu Lys Pro Phe Glu Arg Asp Ile Ser Thr Glu Ile Tyr
    130                 135                 140

Gln Ala Gly Ser Thr Pro Ser Asn Gly Val Glu Gly Phe Asn Ser Tyr
145                 150                 155                 160

Phe Pro Leu Gln Ser Tyr Gly Phe Gln Pro Thr Asn Gly Val Gly Tyr
                165                 170                 175

Gln Pro Tyr Arg Val Val Val Leu Ser Phe Glu Leu Leu His Ala Pro
            180                 185                 190

<210> 3
<211> 576
<212> DNA

<213>  Artificial Sequence

<220>
<223>  Genetic coding for SEQ ID No 1

<400>  3
ccgaacatta ccaacctgtg cccgtttggc gaagtgttta acgcgacccg ctttgcgagc      60

gtgtatgcgt ggaaccgcaa acgcattagc aactgcgtgg cggattatag cgtgctgtat     120

aacagcgcga gctttagcac ctttaaatgc tatggcgtga gcccgaccaa actgaacgat     180

ctgtgcttta ccaacgtgta tgcggatagc tttgtgattc gcggcgatga agtgcgccag     240

attgcgccgg gccagaccgg caaaattgcg gattataact ataaactgcc ggatgatttt     300

accggctgcg tgattgcgtg gaacagcaac aacctggata gcaaagtggg cggcaactat     360

aactatctgt atcgcctgtt tcgcaaaagc aacctgaaac cgtttgaacg cgatattagc     420

accgaaattt atcaggcggg cagcaccccg tgcaacggcg tggaaggctt taactgctat     480

tttccgctgc agagctatgg ctttcagccg accaacggcg tgggctatca gccgtatcgc     540

gtggtggtgc tgagctttga actgctgcat gcgccg                               576


<210>  4
<211>  576
<212>  DNA
<213>  Artificial Sequence

<220>
<223>  Genetic coding for SEQ ID No 2

<400>  4
ccgaacatca ccaacctgtc tccgttcggt gaagttttca acgcgacccg tttcgcgtct      60

gtttacgcgt ggaaccgtaa acgtatctct aactctgttg cggactactc tgttctgtac     120

aactctgcgt ctttctctac cttcaaatct tacggtgttt ctccgaccaa actgaacgac     180

ctgtctttca ccaacgttta cgcggactct ttcgttatcc gtggtgacga agttcgtcag     240

atcgcgccgg gtcagaccgg taaaatcgcg gactacaact acaaactgcc ggacgacttc     300

accggttctg ttatcgcgtg gaactctaac aacctggact ctaaagttgg tggtaactac     360

aactacctgt accgtctgtt ccgtaaatct aacctgaaac cgttcgaacg tgacatctct     420

accgaaatct accaggcggg ttctaccccg tctaacggtg ttgaaggttt caactcttac     480

ttcccgctgc agtcttacgg tttccagccg accaacggtg ttggttacca gccgtaccgt     540

gttgttgttc tgtctttcga actgctgcac gcgccg                               576


<210>  5
<211>  1273
<212>  PRT
<213>  SARS-CoV-2 virus

<400>  5

<210> 6
<211> 1273
<212> PRT
<213> SARS-CoV-2 virus

<400> 6

Met Phe Val Phe Leu Val Leu Leu Pro Leu Val Ser Ser Gln Cys Val
1               5                   10                  15

Asn Leu Thr Thr Arg Thr Gln Leu Pro Pro Ala Tyr Thr Asn Ser Phe
            20                  25                  30

Thr Arg Gly Val Tyr Tyr Pro Asp Lys Val Phe Arg Ser Ser Val Leu
        35                  40                  45

His Ser Thr Gln Asp Leu Phe Leu Pro Phe Phe Ser Asn Val Thr Trp
    50                  55                  60

Phe His Ala Ile His Val Ser Gly Thr Lys Gly Thr Lys Arg Phe Asp
65                  70                  75                  80

Asn Pro Val Leu Pro Phe Asn Asp Gly Val Tyr Phe Ala Ser Thr Glu
                85                  90                  95

Lys Ser Asn Ile Ile Arg Gly Trp Ile Phe Gly Thr Thr Leu Asp Ser
            100                 105                 110

Lys Thr Gln Ser Leu Leu Ile Val Asn Asn Ala Thr Asn Val Val Ile
        115                 120                 125

Lys Val Cys Glu Phe Gln Phe Cys Asn Asp Pro Phe Leu Gly Val Tyr
    130                 135                 140

Tyr His Lys Asn Asn Lys Ser Trp Met Glu Ser Glu Phe Arg Val Tyr
145                 150                 155                 160

Ser Ser Ala Asn Asn Cys Thr Phe Glu Tyr Val Ser Gln Pro Phe Leu
                165                 170                 175

Met Asp Leu Glu Gly Lys Gln Gly Asn Phe Lys Asn Leu Arg Glu Phe
            180                 185                 190

Val Phe Lys Asn Ile Asp Gly Tyr Phe Lys Ile Tyr Ser Lys His Thr
        195                 200                 205

Pro Ile Asn Leu Val Arg Asp Leu Pro Gln Gly Phe Ser Ala Leu Glu
    210                 215                 220

Pro Leu Val Asp Leu Pro Ile Gly Ile Asn Ile Thr Arg Phe Gln Thr
225                 230                 235                 240

Leu Leu Ala Leu His Arg Ser Tyr Leu Thr Pro Gly Asp Ser Ser Ser
                245                 250                 255

Gly Trp Thr Ala Gly Ala Ala Ala Tyr Tyr Val Gly Tyr Leu Gln Pro
            260                 265                 270

Arg Thr Phe Leu Leu Lys Tyr Asn Glu Asn Gly Thr Ile Thr Asp Ala
        275                 280                 285

Val Asp Cys Ala Leu Asp Pro Leu Ser Glu Thr Lys Cys Thr Leu Lys
    290                 295                 300

Ser Phe Thr Val Glu Lys Gly Ile Tyr Gln Thr Ser Asn Phe Arg Val
305                 310                 315                 320

Gln Pro Thr Glu Ser Ile Val Arg Phe Pro Asn Ile Thr Asn Leu Cys
                325                 330                 335

Pro Phe Gly Glu Val Phe Asn Ala Thr Arg Phe Ala Ser Val Tyr Ala
            340                 345                 350

Trp Asn Arg Lys Arg Ile Ser Asn Cys Val Ala Asp Tyr Ser Val Leu
        355                 360                 365

Tyr Asn Ser Ala Ser Phe Ser Thr Phe Lys Cys Tyr Gly Val Ser Pro
    370                 375                 380

Thr Lys Leu Asn Asp Leu Cys Phe Thr Asn Val Tyr Ala Asp Ser Phe
385                 390                 395                 400

Val Ile Arg Gly Asp Glu Val Arg Gln Ile Ala Pro Gly Gln Thr Gly
                405                 410                 415

Lys Ile Ala Asp Tyr Asn Tyr Lys Leu Pro Asp Asp Phe Thr Gly Cys
            420                 425                 430

Val Ile Ala Trp Asn Ser Asn Asn Leu Asp Ser Lys Val Gly Gly Asn

        435                 440                 445

Tyr Asn Tyr Leu Tyr Arg Leu Phe Arg Lys Ser Asn Leu Lys Pro Phe
    450                 455                 460

Glu Arg Asp Ile Ser Thr Glu Ile Tyr Gln Ala Gly Ser Thr Pro Cys
465                 470                 475                 480

Asn Gly Val Glu Gly Phe Asn Cys Tyr Phe Pro Leu Gln Ser Tyr Gly
                485                 490                 495

Phe Gln Pro Thr Asn Gly Val Gly Tyr Gln Pro Tyr Arg Val Val Val
            500                 505                 510

Leu Ser Phe Glu Leu Leu His Ala Pro Ala Thr Val Cys Gly Pro Lys
        515                 520                 525

Lys Ser Thr Asn Leu Val Lys Asn Lys Cys Val Asn Phe Asn Phe Asn
    530                 535                 540

Gly Leu Thr Gly Thr Gly Val Leu Thr Glu Ser Asn Lys Lys Phe Leu
545                 550                 555                 560

Pro Phe Gln Gln Phe Gly Arg Asp Ile Ala Asp Thr Thr Asp Ala Val
                565                 570                 575

Arg Asp Pro Gln Thr Leu Glu Ile Leu Asp Ile Thr Pro Cys Ser Phe
            580                 585                 590

Gly Gly Val Ser Val Ile Thr Pro Gly Thr Asn Thr Ser Asn Gln Val
        595                 600                 605

Ala Val Leu Tyr Gln Asp Val Asn Cys Thr Glu Val Pro Val Ala Ile
    610                 615                 620

His Ala Asp Gln Leu Thr Pro Thr Trp Arg Val Tyr Ser Thr Gly Ser
625                 630                 635                 640

Asn Val Phe Gln Thr Arg Ala Gly Cys Leu Ile Gly Ala Glu His Val
                645                 650                 655

Asn Asn Ser Tyr Glu Cys Asp Ile Pro Ile Gly Ala Gly Ile Cys Ala
            660                 665                 670

Ser Tyr Gln Thr Gln Thr Asn Ser Pro Arg Arg Ala Arg Ser Val Ala
        675                 680                 685

Ser Gln Ser Ile Ile Ala Tyr Thr Met Ser Leu Gly Ala Glu Asn Ser
    690                 695                 700

Val Ala Tyr Ser Asn Asn Ser Ile Ala Ile Pro Thr Asn Phe Thr Ile
705                 710                 715                 720

Ser Val Thr Thr Glu Ile Leu Pro Val Ser Met Thr Lys Thr Ser Val
                725                 730                 735

Asp Cys Thr Met Tyr Ile Cys Gly Asp Ser Thr Glu Cys Ser Asn Leu
            740                 745                 750

Leu Leu Gln Tyr Gly Ser Phe Cys Thr Gln Leu Asn Arg Ala Leu Thr
        755                 760                 765

Gly Ile Ala Val Glu Gln Asp Lys Asn Thr Gln Glu Val Phe Ala Gln
    770                 775                 780

Val Lys Gln Ile Tyr Lys Thr Pro Pro Ile Lys Asp Phe Gly Gly Phe
785                 790                 795                 800

Asn Phe Ser Gln Ile Leu Pro Asp Pro Ser Lys Pro Ser Lys Arg Ser
                805                 810                 815

Phe Ile Glu Asp Leu Leu Phe Asn Lys Val Thr Leu Ala Asp Ala Gly
            820                 825                 830

Phe Ile Lys Gln Tyr Gly Asp Cys Leu Gly Asp Ile Ala Ala Arg Asp
        835                 840                 845

Leu Ile Cys Ala Gln Lys Phe Asn Gly Leu Thr Val Leu Pro Pro Leu
    850                 855                 860

Leu Thr Asp Glu Met Ile Ala Gln Tyr Thr Ser Ala Leu Leu Ala Gly
865                 870                 875                 880

Thr Ile Thr Ser Gly Trp Thr Phe Gly Ala Gly Ala Ala Leu Gln Ile
                885                 890                 895

Pro Phe Ala Met Gln Met Ala Tyr Arg Phe Asn Gly Ile Gly Val Thr
            900                 905                 910

Gln Asn Val Leu Tyr Glu Asn Gln Lys Leu Ile Ala Asn Gln Phe Asn
        915                 920                 925

Ser Ala Ile Gly Lys Ile Gln Asp Ser Leu Ser Ser Thr Ala Ser Ala
    930                 935                 940

Leu Gly Lys Leu Gln Asp Val Val Asn Gln Asn Ala Gln Ala Leu Asn
945                 950                 955                 960

Thr Leu Val Lys Gln Leu Ser Ser Asn Phe Gly Ala Ile Ser Ser Val
                965                 970                 975

Leu Asn Asp Ile Leu Ser Arg Leu Asp Lys Val Glu Ala Glu Val Gln
            980                 985                 990

Ile Asp Arg Leu Ile Thr Gly Arg  Leu Gln Ser Leu Gln  Thr Tyr Val
        995                 1000                 1005

Thr Gln  Gln Leu Ile Arg Ala  Ala Glu Ile Arg Ala  Ser Ala Asn
    1010                 1015                 1020

Leu Ala  Ala Thr Lys Met Ser  Glu Cys Val Leu Gly  Gln Ser Lys
    1025                 1030                 1035

Arg Val  Asp Phe Cys Gly Lys  Gly Tyr His Leu Met  Ser Phe Pro
    1040                 1045                 1050

Gln Ser  Ala Pro His Gly Val  Val Phe Leu His Val  Thr Tyr Val
    1055                 1060                 1065

Pro Ala  Gln Glu Lys Asn Phe  Thr Thr Ala Pro Ala  Ile Cys His
    1070                 1075                 1080

Asp Gly  Lys Ala His Phe Pro  Arg Glu Gly Val Phe  Val Ser Asn
    1085                 1090                 1095

Gly Thr  His Trp Phe Val Thr  Gln Arg Asn Phe Tyr  Glu Pro Gln
    1100                 1105                 1110

Ile Ile  Thr Thr Asp Asn Thr  Phe Val Ser Gly Asn  Cys Asp Val
    1115                 1120                 1125

Val Ile  Gly Ile Val Asn Asn  Thr Val Tyr Asp Pro  Leu Gln Pro
    1130                 1135                 1140

Glu Leu  Asp Ser Phe Lys Glu  Glu Leu Asp Lys Tyr  Phe Lys Asn
    1145                 1150                 1155

His Thr  Ser Pro Asp Val Asp  Leu Gly Asp Ile Ser  Gly Ile Asn
    1160                 1165                 1170

Ala Ser  Val Val Asn Ile Gln  Lys Glu Ile Asp Arg  Leu Asn Glu
    1175                 1180                 1185

Val Ala Lys Asn Leu Asn Glu Ser Leu Ile Asp Leu Gln Glu Leu
    1190                1195                1200

Gly Lys Tyr Glu Gln Tyr Ile Lys Trp Pro Trp Tyr Ile Trp Leu
    1205                1210                1215

Gly Phe Ile Ala Gly Leu Ile Ala Ile Val Met Val Thr Ile Met
    1220                1225                1230

Leu Cys Cys Met Thr Ser Cys Cys Ser Cys Leu Lys Gly Cys Cys
    1235                1240                1245

Ser Cys Gly Ser Cys Cys Lys Phe Asp Glu Asp Asp Ser Glu Pro
    1250                1255                1260

Val Leu Lys Gly Val Lys Leu His Tyr Thr
    1265                1270

<210> 7
<211> 291
<212> PRT
<213> SARS-CoV-2 virus

<400> 7

Thr Gln Asn Val Leu Tyr Glu Asn Gln Lys Leu Ile Ala Asn Gln Phe
1               5                   10                  15

Asn Ser Ala Ile Gly Lys Ile Gln Asp Ser Leu Ser Ser Thr Ala Ser
            20                  25                  30

Ala Leu Gly Lys Leu Gln Asp Val Val Asn Gln Asn Ala Gln Ala Leu
        35                  40                  45

Asn Thr Leu Val Lys Gln Leu Ser Ser Asn Phe Gly Ala Ile Ser Ser
    50                  55                  60

Val Leu Asn Asp Ile Leu Ser Arg Leu Asp Lys Val Glu Ala Glu Val
65                  70                  75                  80

Gln Ile Asp Arg Leu Ile Thr Gly Arg Leu Gln Ser Leu Gln Thr Tyr
                85                  90                  95

Val Thr Gln Gln Leu Ile Arg Ala Ala Glu Ile Arg Ala Ser Ala Asn
            100                 105                 110

Leu Ala Ala Thr Lys Met Ser Glu Cys Val Leu Gly Gln Ser Lys Arg
        115                 120                 125

Val Asp Phe Cys Gly Lys Gly Tyr His Leu Met Ser Phe Pro Gln Ser
    130                 135                 140

Ala Pro His Gly Val Val Phe Leu His Val Thr Tyr Val Pro Ala Gln
145                 150                 155                 160

Glu Lys Asn Phe Thr Thr Ala Pro Ala Ile Cys His Asp Gly Lys Ala
                165                 170                 175

His Phe Pro Arg Glu Gly Val Phe Val Ser Asn Gly Thr His Trp Phe
            180                 185                 190

Val Thr Gln Arg Asn Phe Tyr Glu Pro Gln Ile Ile Thr Thr Asp Asn
        195                 200                 205

Thr Phe Val Ser Gly Asn Cys Asp Val Val Ile Gly Ile Val Asn Asn
    210                 215                 220

Thr Val Tyr Asp Pro Leu Gln Pro Glu Leu Asp Ser Phe Lys Glu Glu
225                 230                 235                 240

Leu Asp Lys Tyr Phe Lys Asn His Thr Ser Pro Asp Val Asp Leu Gly
                245                 250                 255

Asp Ile Ser Gly Ile Asn Ala Ser Val Val Asn Ile Gln Lys Glu Ile
            260                 265                 270

Asp Arg Leu Asn Glu Val Ala Lys Asn Leu Asn Glu Ser Leu Ile Asp
        275                 280                 285

Leu Gln Glu
    290

<210>  8
<211>  291
<212>  PRT
<213>  SARS-CoV-2 virus

<400>  8

Thr Gln Asn Val Leu Tyr Glu Asn Gln Lys Leu Ile Ala Asn Gln Phe
1               5                   10                  15

Asn Ser Ala Ile Gly Lys Ile Gln Asp Ser Leu Ser Ser Thr Ala Ser
            20                  25                  30

Ala Leu Gly Lys Leu Gln Asp Val Val Asn Gln Asn Ala Gln Ala Leu
        35                  40                  45

Asn Thr Leu Val Lys Gln Leu Ser Ser Asn Phe Gly Ala Ile Ser Ser
    50                  55                  60

Val Leu Asn Asp Ile Leu Ser Arg Leu Asp Lys Val Glu Ala Glu Val
65                  70                  75                  80

Gln Ile Asp Arg Leu Ile Thr Gly Arg Leu Gln Ser Leu Gln Thr Tyr
                85                  90                  95

Val Thr Gln Gln Leu Ile Arg Ala Ala Glu Ile Arg Ala Ser Ala Asn
            100                 105                 110

Leu Ala Ala Thr Lys Met Ser Glu Ser Val Leu Gly Gln Ser Lys Arg
        115                 120                 125

Val Asp Phe Ser Gly Lys Gly Tyr His Leu Met Ser Phe Pro Gln Ser
    130                 135                 140

Ala Pro His Gly Val Val Phe Leu His Val Thr Tyr Val Pro Ala Gln
145                 150                 155                 160

Glu Lys Asn Phe Thr Thr Ala Pro Ala Ile Cys His Asp Gly Lys Ala
                165                 170                 175

His Phe Pro Arg Glu Gly Val Phe Val Ser Asn Gly Thr His Trp Phe
            180                 185                 190

Val Thr Gln Arg Asn Phe Tyr Glu Pro Gln Ile Ile Thr Thr Asp Asn
        195                 200                 205

Thr Phe Val Ser Gly Asn Ser Asp Val Val Ile Gly Ile Val Asn Asn
    210                 215                 220

Thr Val Tyr Asp Pro Leu Gln Pro Glu Leu Asp Ser Phe Lys Glu Glu
225                 230                 235                 240

Leu Asp Lys Tyr Phe Lys Asn His Thr Ser Pro Asp Val Asp Leu Gly
                245                 250                 255

Asp Ile Ser Gly Ile Asn Ala Ser Val Val Asn Ile Gln Lys Glu Ile
            260                 265                 270

Asp Arg Leu Asn Glu Val Ala Lys Asn Leu Asn Glu Ser Leu Ile Asp
        275                 280                 285

Leu Gln Glu

    290

## Description

The invention relates to the technical field of immunogenic compositions, in particular to the use of antigenic moieties in combination with liposomal formulations as an adjuvant. The invention further relates to a method for producing the composition, the composition for use as a medicament and in particular for use as a vaccine.

The concept of provoking a protective immune response in the body by means of vaccines in order to prepare for a future exposure to viruses has been a commonly used means in medicine for a long time. With the outbreak of a novel kind of the highly pathogenic corona viruses, the search for efficient vaccination has become even more urgent. Since the first outbreak of SARS-CoV in 2002, the medical and scientific community demonstrated excellent efforts in the structural analysis and understanding of mechanisms related to corona viruses. Despite these efforts, there are still no treatments or prophylactics available which would reliably mitigate the risks that corona viruses pose to human and animal health.

It is the purpose of the subject matter of this application to overcome the current shortcomings of the art. It is in particular a purpose to provide an immunogenic composition comprising an antigenic moiety and a liposomal formulation which induce an immune response against viruses, in particular viruses of the corona type. It is a further purpose of the present invention to suggest a method of producing the composition, to suggest the composition for use as a medicament and in particular for use as a vaccine.

The invention relates to an immunogenic composition comprising
(a) an antigenic moiety, preferably an antigenic moiety being or comprising an amino acid sequence corresponding to a surface protein domain of SARS-CoV-2 virus; and
(b) a liposomal formulation as an adjuvant.

The invention is based on the idea to use peptides which, at least in sections, are similar to the composition of surface proteins of a virus, such as SARS-CoV-2 virus, as an antigenic moiety in combination with liposomal formulations.

The invention in particular relates to an immunogenic composition as described above, wherein the antigenic moiety (a) is or comprises an amino acid sequence corresponding to
(i) the receptor binding domain RBD of Spike protein S of SARS-CoV-2 virus;
(ii) an immunogenic fragment of the said receptor binding domain RBD;
(iii) a sequence having at least 90%, preferably at least 95%, more preferably at least 98% sequence identity to the receptor binding domain RBD of Spike protein S of SARS-CoV-2 virus.

With regard to SARS-CoV-2 virus, of particular interest is the receptor binding domain RBD of the virus which has a strong binding affinity to a host cell receptor and hence creates a configuration for subsequent fusion of the viral and the cellular membranes.

In the case of corona virus SARS-CoV-2, the RBD forms part of structural spike protein S. In particular, sub-unit S₁ of the transmembrane spike S glycoprotein, that forms homotrimers protruding from the viral surface, is of interest (Walls et al, Structure, Function, and antigenicity of the SARS-CoV-2 Spike Glycoprotein, Cell (2020), https://doi.org/10.1016/ j.cell.2020.02. 058). The receptor binding domain RBD has been found to bind strongly to human and bat angiotensin-converting enzyme 2 (ACE2) receptors and therefore serves as a target for development of vaccines (W Tai et al., Characterization of the receptor-binding domain (RBD) of 2019 novel coronavirus: implication for the development of RBD protein as a viral attachment inhibitor and vaccine; Cell Mol Immunol (2020); https://doi.org/10.1038/s41423-020-0400-4). SARS-CoV-2 Spike S protein is surface exposed on virions an infected cell. S protein confers virion docking to cells and endocytosis as well as release of replication machinery into the cytoplasm. S is the most potent unit of immune attack. S has multiple domains of which the receptor binding domain (RBD) is most exposed. RBD is the most potent point of virus neutralization.

For the purposes of this invention, SARS-CoV-2 virus means the virus as described in detail by Gorbalenya et al. The species Severe acute respiratory syndrome-related coronavirus: classifying 2019-nCoV and naming it SARS-CoV-2. Nat. Microbiol. https://doi.org/10.1038/s41564-020-0695-z (2020). For the purposes of the invention, the receptor binding domain RBD of spike protein S of SARS-CoV-2 virus means the amino acid sequence as deposited in GenBank/SRA (https://www.ncbi.nlm.nih.gov/genbank/sars-cov-2-seqs/; retrieved 06.04.2020). The scope of the invention also extends to an immunogenic fragment thereof or a sequence having at least 90%, preferably at least 95%, more preferably at least 98% sequence identity to the said receptor binding domain RBD.

According to the invention, the immunogenic composition further comprises a liposomal formulation as an adjuvant. The authors have found that a combination of the antigenic moiety and the adjuvant induces an immune response that is not significantly different from the immune response induced by the antigenic moiety alone. The liposomal system, however, enhances the effect of the antigenic moiety by enhancing the immune response. The liposomal system therefore provides for better response throughout the population and/or reduces the dose to be administered / the side effects which might accompany the vaccination.

A further aspect of the invention relates to a composition as described above, wherein the antigenic moiety is or comprises an amino acid sequence selected from
(i) SEQ ID No 1;
(ii) an immunogenic fragment of the said sequence;
(iii) a sequence having at least 90%, preferably at least 95%, more preferably at least 98% sequence identity to said SEQ ID No 1.

By SEQ ID No 1 is meant the receptor binding domain RBD of the Italian variant of the Spike protein S of SARS-CoV-2 virus according to GenBank MT066156.1 (retrieved 04.04.2020). The full Spike protein S of SARS-CoV-2/INMI1/human/2020/ITA virus has the protein sequence listed under SEQ ID 5 (ITA).

The receptor binding domain (SEQ ID 1) forms part thereof. The receptor binding domain corresponds to amino acids 329 to 521 of the whole protein sequence as described in the reference database (https://www.ncbi.nlm.nih.gov/genbank/sars-cov-2-seqs/) .

By SEQ ID No 1 is also meant the receptor binding domain RBD of the Chinese variant of the Spike protein S of SARS-CoV-2 virus according to GenBank MT226610.1 (retrieved 06.04.2020). The Spike protein S of SARS-CoV-2/KMS1/human/2020/CHN virus has the protein sequence as provided under SEQ ID 6 (CHN).

The receptor binding domain (SEQ ID 1) forms part thereof. The receptor binding domain corresponds to amino acids 329 to 521 of the whole protein sequence as described in the reference database (https://www.ncbi.nlm.nih.gov/genbank/sars-cov-2-seqs/). In this case the sequence of the RBD (SEQ ID 1) for both the Italian and Chinese Variant are identical. However, modifications are possible and well within the scope of this invention.

The skilled person will appreciate that modifications of the sequence may be possible or even desirable. While in some cases, exact structural similarity, e.g. accurate folding of the protein attached to a liposome's surface, is necessary for the immunogenic response, in other cases, deviations are well acceptable. For example, cysteine in the peptide chain may be replaced in part or in full by serine in view of the expression system, which might e.g. be chosen to be an *E.coli* host organism. Disulphide bonds and, accordingly, folding of certain protein domains can sometimes be dispensed with for the purposes of a vaccine. In this case, the RBD used as an antigenic moiety may have the sequence SEQ ID 2 (Cys > Ser replacements).

One aspect of the invention relates to an immunogenic composition as described above, wherein the antigenic moiety (a) is or comprises an amino acid sequence corresponding to
(i) the HR domain of S₂ subunit of spike protein S of SARS-CoV-2 virus;
(ii) an immunogenic fragment of the said receptor binding domain RBD;
(iii) a sequence having at least 90%, preferably at least 95%, more preferably at least 98% sequence identity to the receptor binding domain RBD of Spike protein S of SARS-CoV-2 virus.

By HR domain (the postfusion core) of S₂ subunit is meant an amino acid sequence (AAs 912 to 1202) which forms part of structural spike protein S of SARS-Cov-2 virus, in particular, in sub-unit S₂ of the transmembrane spike S glycoprotein. This section has turned out to be characteristic, highly preserved and therefore serves as a target for development of vaccines. The natural sequence of the polypeptide corresponds to the one as deposited in GenBank/SRA (https://www.ncbi.nlm.nih.gov/genbank/sars-cov-2-seqs/; retrieved 06.04.2020) and is given as SEQ ID 7.

The scope of the invention, however, also extends to an immunogenic fragment thereof or a sequence having at least 90%, preferably at least 95%, more preferably at least 98% sequence identity to the said receptor binding domain RBD. For example, the cysteins may be replaced by serine, hence giving a sequence of the HR domains of S₂ subunit (AAs 912 to 1202) as listed in SEQ ID 8.

HR1 and HR2 are the post fusion core of the spike S protein and are located on the spike S₂ subunit. Similar to severe acute respiratory syndrome (SARS)-CoV, SARS-CoV-2 has the ability to utilize human angiotensin-converting receptor (ACE2) as a target to infect human cells. Spike (S) protein S2 subunit plays a central role in mediating virus fusion with the host cell, in which the heptad repeat 1 (HR1) and heptad repeat 2 (HR2) can interact to form six-helical bundle, thereby bringing viral and cellular membranes in close proximity for fusion.

Good results can be obtained, if the antigenic moiety and the liposomal formulation are administered to the patient as a mixture. A further aspect of the invention, however, relates to a composition as described above, wherein the antigenic moiety is linked to the liposomal surface to give a proteoliposome.

The authors have found that particularly good results can be achieved if particles are provided that are similar to the actual virus. When liposomes are used as a carrier for the antigenic moiety to give a proteoliposome, such liposomes mimic the virus in size and composition. The surface proteins are presented in a way inspired by the virus' structure and are more likely to produce specific antibodies and cellular immunity against immunodominant epitopes of the virus.

The antigenic moiety may be linked to the liposomal surface:
- covalently, in particular by means of an EDC (1-ethyl-3-(3-dimethylaminopropyl)carbodiimide) crosslinker;
- electrostatically, in particular by means of negatively charged functional groups present on the outer surface of the liposomal bilayer.
Amongst these two, electrostatic linking can be obtained in a more straightforward production process.

For example, covalent attachment of the antigenic moiety to the liposomal surface can be achieved through a EDC (1-ethyl-3-(3-dimethylaminopropyl)carbodiimide) crosslinker. Such a crosslinker can react with carboxyl groups on the one hand and amino groups on the other hand to form amide bonds. Electrostatic interactions can be formed by using outer membrane lipids having a charge, for example phosopholipids, such as DSPE (1,2-Distearoyl-*sn*-glycero-3-phosphoethanolamine). The negatively charged heads of such phospholipids interact with positively charged proteins.

Another aspect of the invention relates to a composition as described above, wherein the antigenic moiety is anchored in the liposomal surface, in particular by means of secondary protein structures linked to the antigenic moiety. Such secondary protein structure may be a structure which exposes apolar groups towards the liposomal bilayer, in particular a α-helix of a protein. It is an advantage of the said anchoring that the secondary protein structure can be provided directly upon expression of the antigenic moiety, in particular the RBD, that it inserts reliably into to the lipid bilayer, and that subsequent surface modification steps may be minimized.

In a preferred embodiment, the lipid bilayer of the lipids in the liposomal formulation substantially consists of cholesterol, phospholipids, in particular phosphatidylcholine and/or phosphatidylglycerol. By the term "substantially consists of" is meant that the components together add up to a total of at least 80 wt-%, preferably at least 90 wt-%, more preferably at least 95 wt% of the lipid bilayer. Examples of phosphatidylcholine include DOPC and/or HSPC. Examples of phosphatidylglycerol include DSPG. The skilled person will acknowledge, that alternatives thereof may be used with similar effects and that variations in relative amounts are possible. However, the authors have found that relative amounts of approximately 30 to 50 wt-% of cholesterol (with regard to the total amount of liposome components) combined with approximately 50 to 70 wt-% of a phosphatidylcholine and phosphatidylglycerol mixture (with regard to the total amount of liposome components; in a wt-ratio of to 2:1 to 5:1) is particularly suitable to build a basis of the liposome.

The liposomes may be modified by PEGylation. By "PEGylation" is meant polyethylene glycol-modification. This may be performed as a post-modification or pre-modification method. Traditionally, phosphatidylcholine, cholesterol and PEG-lipid are dissolved in the same mixture to yield crude liposomes which are downsized later on by extrusion. This method is called the pre-modification method. In contrast, when applying the post-modification method, bare liposomes composed of phosphatidylcholine and cholesterol are prepared in solvent and are then extruded through suitable membrane(s) and/or are-which is preferred - formed by sonication. Only after the steps of liposome formation/minimization is a PEG-lipid added, preferably in aqueous solution. The method is described in detail in Nakamura, K.; Comparative studies of polyethylene glycol-modified liposomes prepared using different PEG-modification methods; Biochim Biophys Acta, 1818 (2012) 2801-2807. A preferred amount of PEG-lipid in the total weight of the liposomes would range between 0.5 and 5 wt-%.

It is preferred that the liposome additionally comprises at least one more adjuvant compound having a lipophilic moiety, preferably Saponin (QuilA or QS-21), Mannide mono oleate (MMO), monophosphoryl lipid A derived motive from lipopolysaccharide (MPLA LPS) or any combination thereof. While cholesterol and phospholipids account for stability of the formed liposomes and adjuvant activity, especially when injected subcutaneously or intramuscularly, Saponin, MMO and/or MPLA LPS may be added for their excellent immunostimulatory properties in vaccines for humans and animals. The adjuvant compounds having a lipophilic moiety may be added in small amounts of < 3 wt-% preferably < 2 wt-% more preferably <1 wt-% of the overall liposomal composition. It is the purpose of these adjuvants to stimulate the immune response, while being physiologically well tolerated, in contrast to traditionally used adjuvants which were often based on aluminum and suspected to be toxic.

In an embodiment of the invention, where the antigenic moiety is covalently linked to the liposomal surface to give a proteoliposome, the covalent bond between the liposome and the antigenic moiety may been derived from lipid bilayer components having reactive head moieties, preferably DSPE or stearic acid. In this case, DSPE and/or stearic acid are incorporated into the liposomal bilayer in an amount of approximately 1 to 5 wt-% of the total liposome components. Such reactive heads, comprising carboxyl groups or amino groups respectively, may react for example via an EDC crosslinker with a complementary amino group / carboxyl group of the antigenic peptide, to eventually, form an amide bond.

In an embodiment of the invention, where there is an electrostatic interaction between the liposome and the antigenic moiety, the interaction may be established due to lipid bilayer components having charged head moieties. Examples of lipid bilayer components having charged head moieties include DSPG (1,2-Distearoyl-sn-glycero-3-phosphoglycerol) and DOTAP (1,2-dioleoyl-3-trimethylammonium-propane). The choice of charged head moiety depends on the isoelectric point of protein and the pH with which the composition is applied. The RBD of Spike S protein has a theoretical pI of 8.67 and thus at pH 6.8 to 7.0 carries a positive charge. There might be a need for a negatively charged lipid for the specific conditions. Other domains of interest, such as the core protein domain of Spike S2 might have a different pI (e.g. 5.4) and will thus carry a negative charge at a pH between 6.8 and 7.0. In such a case, a positively charged lipid head will be preferred.

If we add a Spike S2 core protein domain as additional option: this one as a theoretical pI of 5.4 and thus is negative at pH 6.8 to 7.0, so the need of a positively charged lipid such as DOTAP

It is preferred that the liposomes of the formulation as described above have a mean diameter between 10 and 120 nm, preferably between 20 and 100 nm, measured by dynamic light scattering DLS. Such sizes best mimic the size of the template corona virus. The morphology and size of the liposomes of the formulation can be varied according to the teachings of EP application No. 19181524.0, the contents of which are included herein by reference.

A further aspect of the invention relates to a method of producing an immunogenic composition as described above, comprising the step of combining
(a) the antigenic moiety and
(b) the liposomal formulation.
As described above, the components may be combined by admixing or mixing. However, the components may also be attached to each other, either covalently or electrostatically or by means of lipophilic interaction. The preferred liposomal compositions and exemplary linking mechanisms have been described above.

It is preferred that, prior to combining (a) the antigenic moiety and (b) the liposomal formulation, there are steps of
- providing lipids in a mixture of organic solvent and an aqueous liquid,
- sonicating to enable liposome formation.
Producing the liposomes by means of sonication, is a simple and reliable way of obtaining liposomes which are less polydisperse, more stable and less prone to degradation than liposomes obtainable by conventional techniques, in particular liposomes obtainable by extrusion techniques. An excellent method for providing high quality liposomes has been described in EP application No. 19181524.0, the contents of which are included herein by reference.

It is preferred that in the method as described above, prior to combining (a) the antigenic moiety and (b) the liposomal formulation, there are performed the steps of:
- providing a gene sequence encoding for
   (i) the receptor binding domain RBD of Spike protein S of SARS-CoV-2 virus;
   (ii) an immunogenic fragment of the said receptor binding domain RBD;
   (iii) a sequence having at least 90%, preferably at least 95%, more preferably at least 98% sequence identity to the receptor binding domain RBD of Spike S protein of SARS-CoV-2 virus;
- expressing the genetic sequence in an expression host organism to give (a) the antigenic moiety.

A gene sequence encoding for the receptor binding domain RBD of Spike protein S of SARS-CoV-2 virus, in particular amino acids 329 to 521, may be provided as SEQ ID 3 (Gene Sequence encoding for SEQ ID 1).

The skilled person will appreciate that variations are well possible, even likely and desirable. The sequence may provide for modifications such as Cys > Ser replacements. In this case, the sequence might be provided as SEQ ID 4 (Gene Sequence Encoding for SEQ ID 2).

The genetic code may be chosen to improve efficiency of expression or may be to optimized in view of the respective expression host organism. The genetic code may be provided on plasmid.

A preferred expression host organism may be *E.coli.* Expression in *E.coli* is well established, efficient and sufficiently ensures sequence mediated immunoresponses. However, other preferred expression host organisms include baculovirus, yeast, mammal cells or insect cells which might better provide for glycosylation, cysteine expression and, as a result, protein folding for a structural and sequence mediated immune response.

After expression the amino acid sequence may be purified by chromatographic methods, e.g. 8x His-Tag affinity chromatography, and the His-Tags may then be removed at previously introduced TEV-cleavage sites.

The invention further relates to an immunogenic composition as described above for use as a medicament. In particular, the immunogenic composition is for use as a vaccine in the prevention of COVID-19. In the latter case, one vaccine shot may preferably comprise an amount of 10 to 100 µg of protein sequence.

The invention will be better understood by means of the following examples. They are not meant, however, to limit the scope of the invention.

### Example 1: Expression of an antigenic moiety

A clone comprising SEQ ID 4 was provided in a plasmid, added into Bacteria BL21 *E. Coli* strain, and expressed to obtain a sufficient peptide amount. The bacteria were collected by centrifugation and subject to lysis. The expressed protein was purified by ion exchange or affinity chromatography (His Tag 8x), the His-Tags were removed by means of TEV protease, followed by an additional purification step based on size exclusion chromatography.

### Example 2: Preparation of a liposomal formulation for admixing with the antigenic moiety

40 mol% Cholesterol and 60 mol% HSPC were mixed in prewarmed ethanol prior to being added to aqueous solution (0.9% NaCL 20 mM Hepes pH, 6.8). After dissolving the solvent and aqueous part, the mixture was sonicated for up to 4 hours at a temperature between 60 and 70°C. After liposomes reached an optimal size distribution (polydispersity index PDI of max. 0.12) sonication was stopped and the solution cooled down to 22°C and purified by filtration.

### Example 3: Admixing of antigenic moiety and liposomal formulation

Purified protein according to Example 1 and the liposomal formulation according to Example 2 were added to give a final protein concentration of 20µg/ml and final total lipid concentration of 5mg/ml

### Example 4: Preparation of a liposomal formulation for admixing with the antigenic moiety

30 mol% cholesterol, 66 to 69 mol% HSPC and 1 mol% MMO were mixed and processed to give a liposomal formulation, following the procedure as described in Example 2. Subsequently, 2 mol% DSPE-PEG2000 were added and incorporated by postinsertion method (described in EP application No. 19181524.0). The mixture was heated to 65° for 1h. Purified protein was obtained as described in Example 1. Protein and liposomal formulation were combined by admixing, to give a final protein concentration of 30µg/ml and a final total lipid concentration of 10mg/ml.

### Example 5: Preparation of a liposomal formulation for electrostatically interact with the antigenic moiety

30 mol% cholesterol, 49% mol% HSPC, 20 mol% DSPG, 0.5 mol% saponin Qs-21 and 0.5 mol% MMO were mixed and processed to give a liposomal formulation, following the procedure as described in Example 2. Purified protein was obtained as described in Example 1. Protein and liposomal formulation were combined to give a final protein concentration of 15µg/ml and a final total lipid concentration of 10mg/ml. The pH of the composition is adjusted to physiologic conditions (pH 6.8-7.0) and due to the negative charge of the DSPG and the positive total charge of RBD protein, electrostatic interactions are established.

### Example 6: Preparation of a liposomal formulation for covalent bonding to an antigenic moiety

40 mol% cholesterol, 39% mol% HSPC, 20 mol% stearic acid 1 mol% saponin QS-21 and 0.5 mol% MMO were mixed and processed to give a liposomal formulation, following the procedure as described in Example 2. Purified protein was obtained as described in Example 1. Protein and liposomal formulation were combined, 6 mM EDC-linker was added and the reaction mixture was incubated over night.

## Claims

1. An immunogenic composition comprising
(a) an antigenic moiety, preferably an antigenic moiety being or comprising an amino acid sequence corresponding to a surface protein domain of SARS-CoV-2 virus; and
(b) a liposomal formulation as an adjuvant.

2. An immunogenic composition according to claim 1, wherein the antigenic moiety (a) is or comprises an amino acid sequence corresponding to
(i) the receptor binding domain RBD of Spike protein S of SARS-CoV-2 virus;
(ii) an immunogenic fragment of the said receptor binding domain RBD;
(iii) a sequence having at least 90%, preferably at least 95%, more preferably at least 98% sequence identity to the said receptor binding domain RBD.

3. A composition according to claim 1 or 2, wherein the antigenic moiety is or comprises an amino acid sequence selected from
(i) SEQ ID NO 1;
(ii) an immunogenic fragment of the said sequence;
(iii) a sequence having at least 90%, preferably at least 95%, more preferably at least 98% sequence identity to said SEQ ID No 1.

4. An immunogenic composition according to claim 1, wherein the antigenic moiety (a) is or comprises an amino acid sequence corresponding to
(i) the HR domain of S₂ subunit of spike protein S of SARS-CoV-2 virus;
(ii) an immunogenic fragment of the said receptor binding domain RBD;
(iii) a sequence having at least 90%, preferably at least 95%, more preferably at least 98% sequence identity to the said receptor binding domain RBD;

5. A composition according to any of the preceding claims, wherein the antigenic moiety is linked to the liposomal surface to give a proteoliposome.

6. A composition according to any of the preceding claims, wherein the antigenic moiety is linked to the liposomal surface:
- covalently, in particular by means of an EDC (1-ethyl-3-(3-dimethylaminopropyl)carbodiimide) crosslinker;
- electrostatically, in particular by means of negatively charged functional groups present on the outer surface of the liposomal bilayer.

7. A composition according to claim 5 or 6, wherein the antigenic moiety is anchored in the liposomal surface, in particular by means of secondary protein structures, in particular at least one α-helix, formed by to the antigenic moiety.

8. A composition according to any of the preceding claims, wherein the lipid bilayer of the liposome of the formulation substantially consists of a combination of cholesterol, phospholipids, in particular phosphatidylcholine and/or phosphatidylglycerol.

9. A composition according to claim 8, wherein the composition additionally comprises at least one more adjuvant compound having a lipophilic moiety, preferably, Saponin (QuilA or QS-21), Mannide mono oleate (MMO), monophosphoryl lipid A derived motive from lipopolysaccharide (MPLA LPS) or any combination thereof.

10. A composition according to any of claims 5 to 9 wherein the covalent bond between the liposome and the antigenic moiety has been derived from lipid bilayer components having reactive head moieties, preferably DSPE or stearic acid.

11. A composition according to any of claims 5 to 9 wherein the electrostatic interaction between the liposome and the antigenic moiety is established due to lipid bilayer components having charged head moieties.

12. A method of producing an immunogenic composition according to any of claims 1 to 11, comprising the step of combining
(a) the antigenic moiety and
(b) the liposomal formulation.

13. A method according to claim 12 further, comprising the steps of, prior to combining (a) the antigenic moiety and (b) the liposomal formulation:
- providing lipids in a mixture of organic solvent and an aqueous liquid,
- sonicating to enable liposome formation.

14. A method according to one of claims 12 or 13, comprising the steps of, prior to combining (a) the antigenic moiety and (b) the liposomal formulation:
- providing a gene sequence encoding for
(i) the receptor binding domain RBD of Spike protein S of SARS-CoV-2 virus;
(ii) an immunogenic fragment of the said receptor binding domain RBD;
(iii) a sequence having at least 90%, preferably at least 95%, more preferably at least 98% sequence identity to the receptor binding domain RBD of Spike protein S of SARS-CoV-2 virus;
- expressing the genetic sequence in an expression host organism to give (a) the antigenic moiety.

15. An immunogenic composition according to one of claims 1 to 11 for use as a medicament.

16. An immunogenic composition according to one of claims 1 to 11 for use as a vaccine in the prevention of COVID-19.
